# EUROPEAN PATENT APPLICATION

(11) **EP 0 950 378 A1**
(43) Date of publication of application: **20.10.1999**
(21) Application number: 99107170.5
(22) Date of filing: 13.04.1999
(51) Int. Cl.: A61B 17/41

(54) **Hair removal electrolysis apparatus**

(30) Priority: 16.04.1998 CA 2231383
(71) Applicant: SILHOUET-TONE (EUROPE) S.A., 1295 Mies (CH)
(72) Inventor: Ghedin, Alphonse, Brossard, Québec JAX 2A5 (CA)
(74) Representative: Kovacs, Paul

(57) **Abstract**

This hair removal electrolysis apparatus consists in a handle, a needle head assembly and a needle protective cover. The handle has an electrically conductive plug connectable to an electric supply forming part of the electrolysis apparatus. The needle head assembly has a head cap and a needle assembly. The head cap can externally fit onto the plug. The needle assembly has a connecting pin that can be electrically connected to the plug and thus to the electric supply, and a needle sized to be inserted into the hair follicle of a patient along side a hair shaft in the dermis. The protective cover is sized and shaped to snugly fit onto the head cap in order to cover the needle and thus to avoid inadvertent breaking of the needle during stockage or accidental stinging during handling. The head cap and the handle are detachably connectable to each other by screwing, clipping or a bayonet type mount. As a result, one may connect the head cap onto the handle without having to remove the protective cover which then acts as a handling device, and subsequently remove the needle head assembly from the handle with the same protective cover which then acts as a safety device.

## Description

The present invention relates to the combination of an handle, a needle head assembly and a protective cover for use in an hair removal electrolysis apparatus.

In the field of permanent hair removal equipment, there are actually three main types of handle and needles systems which can be classified as free needles, refined free needles and needle heads.

Free needles are free and delicate components to be manipulated with tweezers. Most handles adapted to free needles comprise a separate molded tip used to tighten radial slot of the plug of the handle on the needle itself. The molded tip can be a reusable one which must be sterilised between each use, or a disposable one.

Refined free needles use axial spring forces to enhance the electrical contact between the needle and the plug. However, these refined needles are dangerous because the tip may accidentally detached and the needle is propelled by the spring, eventually causing injury to the operator.

The third type of handle and needles system, such as described in US 4,785,808 (Cary), has a fine metallic wire, molded-in inside a threaded plastic head and a cap to protect the needle. This system uses no steel shaft and it does not provide a great electric contact between the needle and the handle. It also does not connect the needle shaft to the handle tightly and securely.

It is known that the electrolysis process may entail contact with the patient and/or operator blood during insertion of the needle alongside the hairshaft in the dermis of the patient, or inadvertent hinging when the operator removes the hair from the dermis with tweezers or fixes the magnifying glass or other manipulation of the type or inadvertent movement of the patient.

Everyone involved with electrolysis, whether the patient or the operator, is getting more and more concerned with the risks of injury and blood transmittable diseases such as AIDS, Hepatitis, etc. Patients are afraid to get a disease from the electrolysis operator or from the previous patients and the electrolysis operator is afraid to get a disease from any of his/ her patients. For the electrolysis operator, the moment where he/she puts the needle on the handle or off the handle is particularly dangerous.

Accordingly, it would be useful to provide a sterile needle head assembly which can be quickly and safely installed on the handle without having to remove the protective cover which then acts as a handling means, or quickly and safely removed from the handle without having to remove the protective cover which then acts as a safety means for stockage.

It would further be useful to provide a needle head assembly that can be installed on the handle or remove from the handle, by hand, without need of tweezers.

It would also be useful to provide a needle bead assembly with a good electrical contact between the needle and the electric supply of the electrolysis apparatus.

### SUMMARY OF THE INVENTION

A first object of the present invention is to provide an apparatus of very specific construction, which satisfies the above mentioned needs in the hair removal electrolysis field.

More particularly, the first object of the invention is to provide an apparatus wherein the needle head assembly can be quickly and safely inserted or removed from the handle, by hand, without need of tweezers, while being covered by the protective cover.

Another object of the invention is to provide an apparatus of the above type wherein a good electrical contact is achieved between the needle and the electrical supply forming part of the electrolysis apparatus.

In accordance with the invention, these objects are achieved with the following combination of an handle, a needle head assembly and a needle protective cover for use in a hair removal electrolysis apparatus.

The handle comprises a sleeve, a plug and a wire. The sleeve is elongated and electrically insulated and it has a main axis and two opposite ends. The plug is electrically conductive and is coaxial with the sleeve. It projects from one of the opposite ends of the sleeve and has an axial hole. The wire is electrically conductive and extends from the plug through the sleeve. It projects out from the other end of the sleeve for connection to an electric supply forming part of an electrolysis apparatus.

The needle head assembly comprises a head cap and a needle assembly. The head cap has an open cavity sized and is shaped to receive the plug. As a result, the head cap can externally fit onto the plug of the handle, or alternatively, on the handle itself. The needle assembly is fixed to the head cap. It comprises a connecting pin extending within the cavity of the head cap so as to snugly fit into the hole of the plug and thus to be electrically connected to the electric supply. The needle assembly also comprises a needle axially projecting from the connecting pin outwards the head cap. The needle is sized to be inserted into the hair follicle of a patient along side a hair shaft in the dermis, so that electrical current can be dispensed through the needle below the surface of the skin, alongside a hair shaft, in the dermis, right up to the hair bulb.

The protective cover is sized and shaped to snugly fit onto the head cap in order to cover the needle projecting axially out of the head cap and thus to avoid inadvertent breaking of the needle during stockage or accidental stinging during handling.

In accordance with the invention, first and second attachment means are provided on the head cap and the handle, respectively. These attachment means are operatively connectable to each other, in order to detachably connect from each other.

As a result, one may easily install the needle head assembly onto the handle without having to remove the protective cover which then acts as a handling means, and subsequently remove the needle head assembly from the handle with the same protective cap which then acts as a safety means.

The attachment means may be of any type provided that they allow detachable connection of the head cap onto the handle by means of the protective cover. These attachment means may be, for example, a screw type mount, a bayonet type mount or a clip-on type mount.

Preferably, the plug on the handle has an axial slot extending through its axial hole. This slot facilitates insertion of the connecting pin of the needle head assembly into the plug and provides a better electrical contact between the pin and the plug.

Preferably also, the needle head assembly and protective cover are of the disposable type.

Thus, the invention provides the combination of a handle, a needle head assembly and a protective cover which makes it unlikely not to say impossible to have a transmission of disease via the human blood.

The invention also lies in the needle head assembly as such, which is of a disposable type and can fit on already existing handles. This needle head assembly comprises a head cap and a needle assembly. The head cap has an open cavity sized and is shaped to receive the plug of the handle, whereby said head cap can externally fit onto the handle, preferably onto the plug of the handle. The needle assembly is fixed to the head cap. It comprises a connecting pin extending within the cavity of the head cap so as to snugly fit into the hole of the plug and thus to be electrically connected to the electric supply. It also comprises a needle axially projecting the connecting pin outwards the head cap. The needle is sized to be inserted into the hair follicle of a patient along side a hair shaft in the dermis. The needle projecting axially out of the head cap can be protected by the protective cover sized and shaped to snugly fit onto the head cap and thus to avoid inadvertent breaking of the needle during stockage or accidental stinging during handling.

The invention and its advantages will be better understood by reading the following non-restrictive description of four preferred embodiments thereof, made with reference to the accompanying drawings.

### BRIEF DESCRIPTION OF THE DRAWINGS

Fig. 1 is an exploded side elevational view of the combination of a handle, a needle head assembly and a needle protective cover according to a first preferred embodiment of the present invention wherein the needle head assembly is connected to the plug of the handle by screwing;
Fig. 2 is an enlarged view of the tip of the handle, the needle head assembly and a needle protective cover shown in Fig. 1, the protective cover being illustrated in cross-section;
Fig. 3a is a side elevational view of the tip of the handle and the needle head assembly shown in Fig. 1, wherein the needle head assembly is illustrated in cross-section;
Fig. 3b is an end view of the tip of the handle shown in Fig.3a;
Fig. 4 is a side elevational view similar to the one of Fig. 3a, which shows a second preferred embodiment of the invention, wherein the needle head assembly is connected onto the plug of the handle with a bayonet type mount;
Fig. 5a is a side elevational view similar to the one of Fig. 3a, which shows a third preferred embodiment of the invention, wherein the needle head assembly is connected to the sleeve of the handle with a clip-on type mount;
Fig. 5b is a end view of the needle head assembly shown in Fig. 5a;
Fig. 6 is a side elevational view similar to the one of Fig. 3a, which shows a fourth preferred embodiment of the invention, wherein the needle head assembly is connected to the plug of the handle with a snap-on type mount;
Fig.7 is a side elevational view of the needle head assembly and the handle of a variant of the fourth preferred embodiment of the invention shown in Fig. 6, wherein the electrically conductive wire extends from the side of the handle, the needle head assembly being shown in cross-section; and
Fig. 8 is a side elevational view of a fifth preferred embodiment of the invention, wherein the needle head assembly is connected onto the handle by screwing.

### DESCRIPTION OF FIVE PREFERRED EMBODIMENTS OF THE INVENTION

As aforesaid, the invention lies in the combination of a handle, a sterile needle head assembly and a needle protective cover, which is devised to allow the installation of the needle head assembly onto the handle by hand, without need of tweezers, and without having to remove the protective covering, thereby reducing the risk of damaging the needle or getting sting by it.

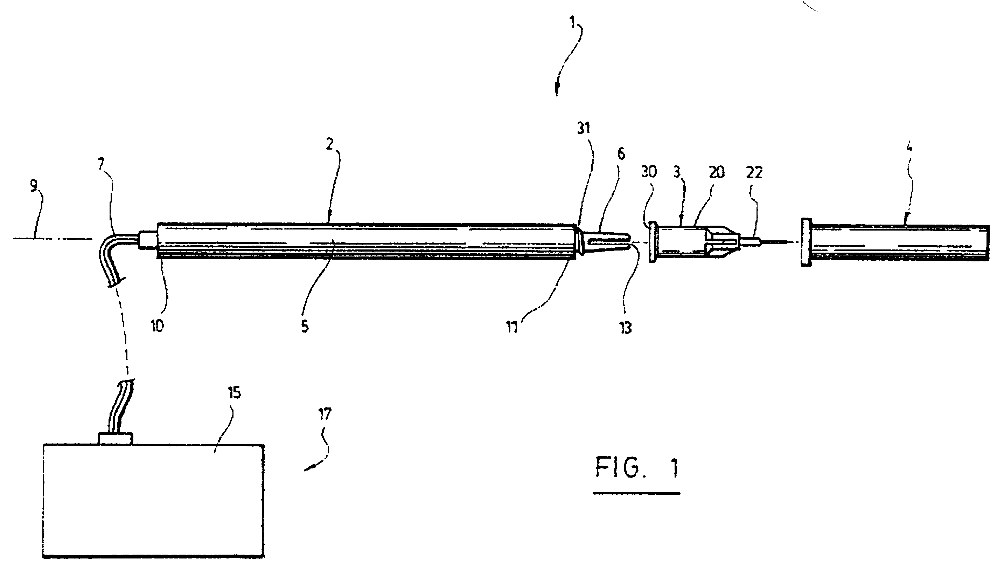

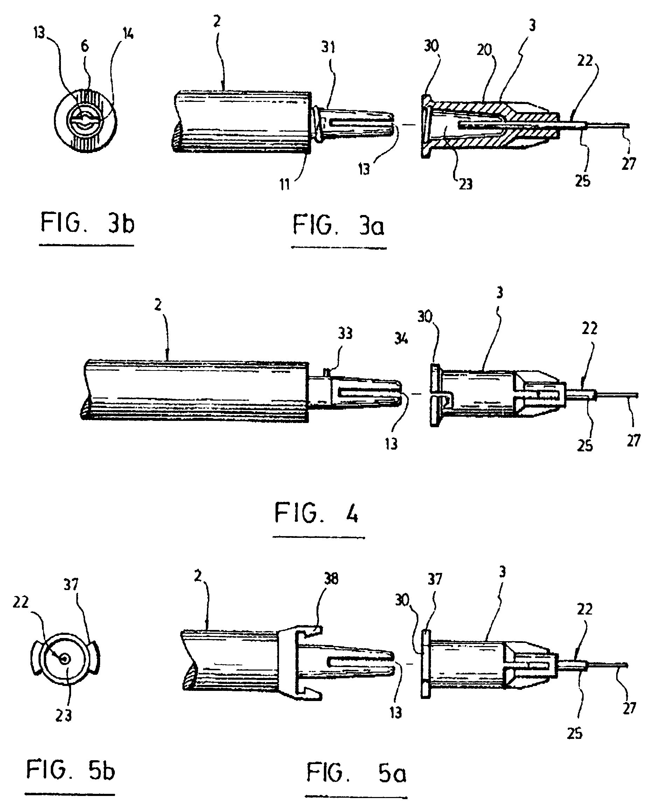

The combination 1 according to a first preferred embodiment of the invention is shown in Fig. 1. It comprises a handle 2 having an elongated electrically insulated sleeve 5 with a smooth outer surface, an electrically conductive plug 6 and an electrically conductive wire 7. The insulated sleeve 5 has a main axis 9 and two opposite ends 10 and 11. The electrically conductive plug 6 is coaxial with the main axis 9 of the sleeve 5 and has an axial hole 13 and a radial slot 14 (see Fig. 3a). It is rigidly attached to the end 11 of the sleeve and has a base portion 31 adjacent to this one end 11. The electrically conductive wire 7 extends from the plug 6 through the sleeve 5 and projects out from the opposite end 10 of the sleeve 5 for connection to an electric supply 15 forming part of a hair removal electrolysis apparatus 17.

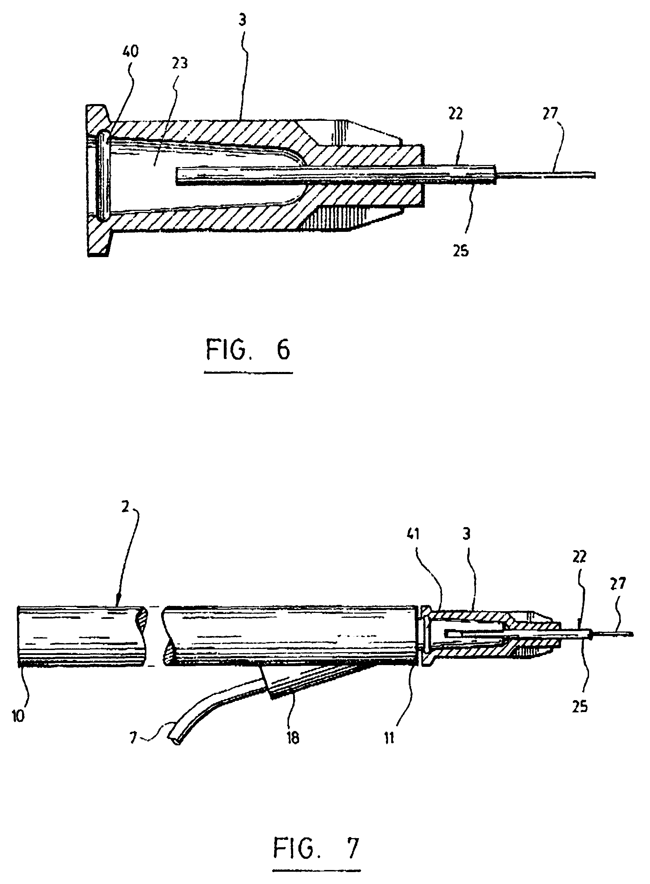

As shown in Fig. 7, the surface of the handle needs not be smooth. As a matter of fact, it may comprise protruding or recessed surfaces to enhance comfort when the handle is held for long periods of time.

Alternatively also, as is also shown in Fig.7, the electrically conductive wire 7 needs not to project out of the end 10 of the sleeve 5 but may rather extend from one side 18 of the handle. This bend of the wire 7 on the handle 2 allows much enhanced comfort during handle manipulation in a working position behind any type of magnifying glass commonly used in this field. The magnifying glass can be held a few inches closer to the patient treated area, when compared to a straight wire connection.

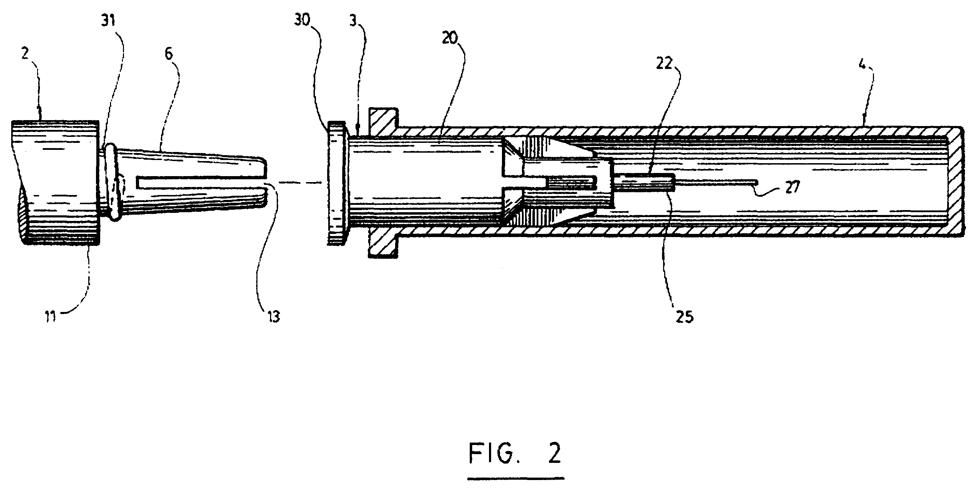

The combination 1 also comprises a needle head assembly 3 comprising a head cap 20 which is made of plastic material, and a needle assembly 22 which is made of a conductive metal. As is better shown in Fig.2, the head cap 20 has an open cavity 23 that is sized and shaped to receive the plug 6. Thus, the head cap 20 can externally fit onto the plug 6. The needle assembly 22 is fixed to the head cap 20 and comprises a connecting pin 25 and a needle 27. The connecting pin 25 extends in the open cavity 23 of the head cap 20 and snugly fits in the hole 13 of the plug 6. The radial slot 14 enables to insert the connecting pin 25 easily in the plug 6 and to maintain a good electrical contact between the pin 25 and the plug 6. The connecting pin 25 is then electrically connected to the electric supply 15 of the hair removal electrolysis apparatus 17. The needle 27 projects axially from the connecting pin 25 outwards the head cap 20 and is sized to be inserted into the hair follicle of a patient along side a hair shaft in the dermis. Electrical current can be dispensed through the needle below the surface of the skin, alongside a hair shaft, in the dermis, until the hair bulb.

Preferably, the size and dimensions of the needle head assembly are preserved in order to make it useable with commonly used handles.

As is better shown in Fig.2, the combination 1 further comprises a protective cover 4 which is made of plastic material and is sized and shaped to snugly fit onto the head cap 20 in order to cover the needle 27 which projects axially out of the head cap 20. The purpose of the protective cover 4 is essentially to avoid inadvertent breaking of the needle 27 during stockage or accidental stinging during handling. The protective cover 4 is fit on the needle head assembly by friction means. The diameter of the open cavity of the protective cover is just a little bit larger than the diameter of the needle head assembly 3.

As aforesaid, the head cap 20 and the protective cover 4 are preferably made of plastic material and the needle head assembly 3 and the protective cover 4 are both of the disposable type.

In accordance with the invention, the above combination of the handle 2, the needle head assembly 3 and the protective cover 4 is improved in that attachment means are provided to detachably connect the needle head assembly to the handle. For this purpose, a first attachment means is provided on the head cap 20 of the needle head assembly 3 and a second attachment means is provided on the plug 6 of the handle 2, or, alternatively, on the handle 2 itself. These attachment means are operatively connectable to each other in order to detachably connect the head cap 20 to the handle 2. As a result, one may install the needle head assembly 3 onto the handle 2 without having to remove the protective cover 4 which then acts as a handling means, and subsequently remove the needle head assembly 3 from the handle 2 with the same protective cover which then acts as a safety means.

In the first, second and third preferred embodiments of the invention shown in Figs. 1 to 4, 6 and 7, the open cavity 23 of the head cap 20 has an internal rim portion 30 on which the first attachment means is mounted. The second attachment means is externally mounted on the base portion 31 of the plug 6.

In the first preferred embodiment shown in Figs. 1 to 3, the first and second attachment means consist of a screw type mount, viz, a set of cooperating threads. Thus, the needle head assembly 3 can be detachably connected to the plug 6 by screwing.

In the second preferred embodiment shown in Fig.4, the first and second attachment means consist of a bayonet type mount. More particularly, the first and second attachments comprise one or more locking pin 33 projecting radially outwardly from the base 31 of the plug 6 and a socket 34 mounted on the needle head assembly 3. Alternatively, the pin(s) could be mounted on the needle head assembly 3 so as to project radially inwardly within the open cavity 23 of the head cap and the socket 34 could be mounted on the plug 6.

In the third preferred embodiment of the invention shown in Figs. 6 and 7, the needle head assembly 3 is connected on the plug 6 of the handle with a snap-on type mount like the one used in pens. The open cavity 23 of the head cap 20 comprises a circular recess 40 which is devised and shaped to "receive" a corresponding circular bulge 41 (see Fig. 7) projecting from the base 31 of the plug 6. The "snap-on" strength is selected so that the connection between the recess 40 and bulge 41 is stronger than the friction between the external surface of the head cap of the needle head assembly and the internal surface of the protective cover so that when the operator removes the needle head assembly from the handle, the protective cover stays on the needle head assembly unless he or she pinches the protective cover so hard that the friction between it and the head cap becomes stronger than the snap-on strength.

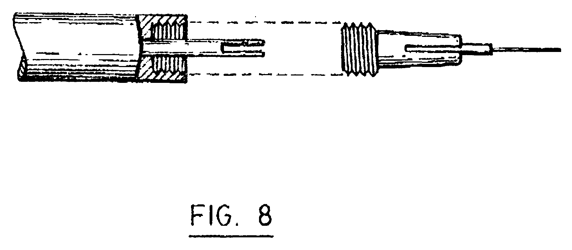

In the fourth and fifth preferred embodiments shown in Figs. 5 and 8, the open cavity 23 of the head cap 20 has an external rim portion 30 on which the first attachment means is mounted. The second attachment means is externally mounted on the handle 2.

In the fourth preferred embodiment shown in Fig. 5, the first and second attachment means consist of clip-on type mount. In this embodiment, the needle head assembly 3 is attached to the handle 2 itself by clipping. The clip-on type mount comprises two pegs 37 and two clippers 38. The pegs 37 are mounted on the external rim portion 30 of the head cap 20 and the dippers 38 are mounted on the external rim portion 31 of the handle 2. Fig. 5a shows a cross section of the pegs 37. Alternatively, the pegs 37 could be mounted on the external rim portion 31 of the handle 2 and the clippers 38 on the external rim portion 30 of the head cap 20. Alternatively also, there could be only one peg and one clipper or more than two pegs and two clippers.

In the fifth preferred embodiment of the invention shown in Fig. 8, the head cap 20 of the needle head assembly 3 has an external rim portion 80 on which is mounted the first attachment means. The second attachment means 81 is mounted on the end 11 of the sleeve 5 of the handle 2. The first and second attachment means consist of a screw type mount, wherein the needle head assembly 3 is attached to the plug 6 by screwing.

It will be obvious for the man skilled in the art that the previously described attachment means are reversible: the second attachment means can be either on the plug of the handle or the handle itself. Also, the attachment means described as the first attachment means (on the needle head assembly) could be "inverted" with the second attachment means (on the handle) or vice-versa.

In use, the needle head assembly is sold with the protective cover already fit on so as to protect the needle from breaking and the operator from stinging. The operator can then hold the protective cover and connect the needle head assembly on the handle by means of the attachment means. When the needle head assembly is firmly attached to the handle, the operator pulls out the protective cover and can start working. When the electrolysis treatment is finished, the operator can put back the protective cover on the needle by pushing it on the head cap and then use this cover as a tool to remove the needle head assembly from the handle, without stinging himself/ herself

Once removed, the needed head assembly can be disposed off together with the protection cover which still "embed" the needle and thus acts as a perfecty device.

All these steps can be caved out by hand, with no tool. Of course, however the distractions could be made to the embodiments disclosed hereinabove without departing from the scope of the invention as defined in the appended claims.

## Claims

1. An hair removal electrolysis apparatus having a handle, a needle head assembly and a needle protective cover, characterised in that:
a) said handle comprises:
• an elongated, electrically insulating sleeve having a main axis and two opposite ends;
• an electrically conductive plug coaxial with said sleeve and projecting from one of said opposite ends, said plug having an axial hole; and
• an electrically conductive wire extending from said plug through said sleeve and projecting out from the other end of said sleeve for connection to an electric supply forming part of said electrolysis apparatus;
b) said needle head assembly comprises:
• a head cap having an open cavity sized and shaped to receive the plug, whereby said head cap can externally fit onto said plug; and
• a needle assembly fixed to the head cap, said needle assembly comprising a connecting pin extending within said cavity so as to snugly fit into the hole of said plug and thus to be electrically connected to said electric supply, and a needle axially projecting from said connecting pin outwards the head cap, said needle being sized to be inserted into the hair follicle of a patient along side a hair shaft in the dermis; and
c) said protective cover is sized and shaped to snugly fit onto the head cap in order to cover the needle projecting axially out of said head cap and thus to avoid inadvertent breaking of said needle during stockage or accidental stinging during handling, wherein first and second attachment means are provided on the head cap and the handle respectively, said first and second attachments being operatively connectable to each other in order to detachably connect said head cap to said handle, whereby one may easily install the needle head assembly onto the plug without having to remove the protective cover which then acts as a handling means, and subsequently remove the needle head assembly from the plug with the same protective cover which then acts as a safety means.

2. The apparatus of claim 1, wherein:
• the open cavity of the head cap of the needle head assembly has an internal rim portion;
• the first attachment means is mounted onto said internal rim portion;
• the plug of the handle has a base portion adjacent to the one end of the sleeve;
• the second attachment means is externally mounted onto said base portion.

3. The apparatus of claim 2, wherein the first and second attachment means consist of a screw type mount, wherein said needle head assembly is attached to said plug by screwing.

4. The apparatus of claim 2, wherein the first and second attachment means consist of a bayonet type mount.

5. The apparatus of claim 4, wherein the bayonet type mount comprises at least one locking pin and a socket, wherein said at least one locking pin is mounted on the needle head assembly and the socket is mounted on the plug.

6. The apparatus of claim 4, wherein the bayonet type mount comprises at least one locking pin and a socket, wherein said at least one locking pin is mounted on the plug and the socket is mounted on the needle head assembly.

7. The apparatus of claim 2, wherein the first and second attachment means consist of a clip-on type mount, wherein said needle head assembly is attached to said plug by clipping.

8. The apparatus of claim 7, wherein the clip-on type mount comprises pegs and clippers, wherein the pegs are mounted on the needle head assembly and the clippers are mounted on the plug.

9. The apparatus of claim 7, wherein the clip-on type mount comprises pegs and clippers, wherein the clippers are mounted on the needle head assembly and the pegs are mounted on the plug.

10. The apparatus of claim 1, wherein:
• the head cap of the needle head assembly has an external rim portion;
• the first attachment means is mounted onto said external rim portion; the second attachment means is mounted on the same end of the elongated sleeve as the plug.

11. The apparatus of claim 10, wherein the first and second attachment means consist of a screw type mount, wherein said needle head assembly is attached to said sleeve by screwing.

12. The apparatus of claim 10, wherein the first and second attachment means consist of a bayonet type mount.

13. The apparatus of claim 12, wherein the bayonet type mount comprises at least one locking pin and a socket, wherein said at least one locking pin is mounted on the needle head assembly and the socket is mounted on the sleeve.

14. The apparatus of claim 12, wherein the bayonet type mount comprises a base and a socket, wherein the base is mounted on the sleeve and the socket is mounted on the needle head assembly.

15. The apparatus of claim 10, wherein the first and second attachment means consist of a clip-on type mount, wherein said needle head assembly is attached to said sleeve by clipping.

16. The apparatus of claim 15, wherein the clip-on type mount comprises pegs and dippers, wherein the pegs are mounted on the needle head assembly and the clippers are mounted on the sleeve.

17. The apparatus of claim 15, wherein the clip-on type mount comprises pegs and clippers, wherein the clippers are mounted on the needle head assembly and the pegs are mounted on the sleeve.

18. The apparatus of anyone of claims 1 to 17, wherein said head cap and protective cover are made of plastic material and said needle head assembly and protective cover are both of the disposable type.

19. The apparatus of anyone of claims 1 to 18, wherein the plug on the handle has an axial slot through the axial hole, whereby the slot enables to insert the connecting pin of the needle head assembly easily in the plug and to maintain a good electrical contact between said pin and the plug.

20. The apparatus of anyone of claims 1 to 19, wherein the handle is connected to an electric supply forming part of an electrolysis apparatus through an electrically conductive wire extending from the side of its sleeve.

21. The apparatus of anyone of claims 1 to 19, wherein the sleeve of the handle comprises protruding surfaces.

22. The apparatus of claim 2, wherein the first and second attachment means consist of a snap-on type mount, wherein the needle head assembly comprises an internal circular recess which fits on the external circular bulge on the plug.

23. The apparatus of claim 2, wherein the first and second attachment means consist of a snap-an type mount, wherein the needle head assembly comprises an internal circular recess which fits on the external circular bulge on the handle.

24. A needle head assembly for use in a hair removal electrolysis apparatus having a handle comprising:
• an elongated, electrically insulating sleeve having a main axis and two opposite ends;
• an electrically conductive plug coaxial with said sleeve and projecting from one of said ends, said plug having an axial hole; and
• an electrically conductive wire extending from said plug through said sleeve and projecting out from the other end of said sleeve for connection to an electric supply forming part of said electrolysis apparatus;
characterises in that said needle head assembly comprises:
• a head cap having an open cavity, sized and shaped to receive the plug, whereby said head cap can be externally fit onto said plug; and
• a needle assembly fixed to the head cap, said needle assembly comprising a connecting pin extending within said cavity so as to snugly fit into the hole of said plug and thus to be electrically connected to said electric supply, and a needle axially projecting from said connecting pin outwards the head cap, said needle being sized to be inserted into the hair follicle of a patient along side a hair shaft in the dermis;
wherein first attachment means are provided on the head cap, said first attachment means being operatively connectable to second attachment means provided on said plug of the handle in order to detachably connect said head cap onto said handle.

25. The assembly of claim 24 wherein
• the open cavity of the head cap of the needle head assembly has an internal rim portion; and
• the first attachment means is mounted onto said internal rim portion.

26. The assembly of claim 25, wherein the first attachment means consists of a screw type mount, wherein said needle head assembly is attached to said plug by screwing.

27. The assembly of claim 25, wherein the first attachment means consists of a bayonet type mount.

28. The assembly of claim 27, wherein the bayonet type mount comprises at least one locking pin and a socket, wherein the socket is mounted on the needle head assembly.

29. The assembly of claim 27, wherein the bayonet type mount comprises at least one locking pin and a socket, wherein said at least locking pin is mounted on the needle head assembly.

30. The assembly of claim 25, wherein the first attachment means consists of a clip-on type mount, wherein said needle head assembly is attached to said handle by clipping.

31. The assembly of claim 30, wherein the clip-on type mount comprises pegs and clippers, wherein the pegs are on the needle head assembly.

32. The assembly of claim 30, wherein the clip-on type mount comprises pegs and clippers, wherein the clippers are on the needle head assembly.

33. The assembly of claim 25 wherein
• the open cavity of the head cap of the needle head assembly has an external rim portion; and
• the first attachment means is mounted onto said external rim portion.

34. The assembly of claim 25, wherein the first attachment means consists of a screw type mount, wherein said needle head assembly is attached to said handle by screwing.

35. The assembly of claim 25, wherein the first attachment means consists of a bayonet type mount.

36. The assembly of claim 35, wherein the bayonet type mount comprises at least one locking pin and a socket, wherein the socket is mounted on the needle head assembly.

37. The assembly of claim 35, wherein the bayonet type mount comprises at least one locking pin and a socket, wherein said at least one locking pin is mounted on the needle head assembly.

38. The assembly of claim 25, wherein the first attachment means consists of a clip-on type mount, wherein said needle head assembly is attached to said handle by clipping.

39. The assembly of claim 38, wherein the dip-on type mount comprises pegs and clippers, wherein the pegs are on the needle head assembly.

40. The assembly of claim 38, wherein the clip-on type mount comprises pegs and clippers, wherein the clippers are on the needle head assembly.

41. The assembly of claim 25, wherein the first attachment means consists of a snap-on type mount, wherein the needle head assembly comprises an internal circular recess.

42. The assembly of anyone of claim 24 to 41, wherein the head cap is made of plastic material and said needle head assembly is of the disposable type.

43. The assembly of anyone of claim 24 to 41, wherein the needle head assembly is covered by a protective cover sized and shaped to snugly fit onto the head cap in order to cover the needle projecting axially out of said head cap and thus to avoid inadvertent breaking of said needle during stockage or accidental stinging during handling.
